# EUROPÄISCHE PATENTSCHRIFT

(11) **EP 1 196 159 B1**
(45) Veröffentlichungstag und Bekanntmachung des Hinweises auf die Patenterteilung: **21.04.2004**
(21) Anmeldenummer: 00954553.4
(22) Anmeldetag: 21.07.2000
(51) Int. Cl.: A61K 31/18, A61P 31/00, A61P 33/00

(54) **VERWENDUNG VON TOSYLCHLORAMID(EN) ZUR BEHANDLUNG VON ERKRANKUNGEN DER HAUT, DER SCHLEIMHAUT, VON ORGANEN UND GEWEBEN**
USE OF TOSYLCHLORAMIDE(S) FOR TREATING DISEASES OF THE SKIN, MUCOUS MEMBRANES, ORGANS AND TISSUES
UTILISATION DE TOSYLCHLORAMIDE(S) POUR LE TRAITEMENT DE MALADIES DE LA PEAU, DE LA MUQUEUSE, D'ORGANES ET DE TISSUS

(30) Priorität: 23.07.1999 DE 19934585
(43) Veröffentlichungstag der Anmeldung: 17.04.2002
(73) Patentinhaber: Rapp, Horst, 75334 Straubenhardt (DE); Heck, Friedbert, 75173 Pforzheim (DE)
(72) Erfinder: Rapp, Horst, 75334 Straubenhardt (DE); Heck, Friedbert, 75173 Pforzheim (DE)
(74) Vertreter: Gehring, Friederike, Dr.
(86) Internationale Anmeldenummer: PCT/EP2000/006997
(87) Internationale Veröffentlichungsnummer: WO 2001/007035

(56) Entgegenhaltungen:
- WO-A-91/07876
- DE-A- 4 137 544
- DE-A- 19 712 565
- US-A- 3 317 540

## Beschreibung

Die Erfindung betrifft die Verwendung von Tosylchloramid(en) zur Herstellung von Arzneimitteln zur Behandlung von Erkrankungen der Haut und der Schleimhaut.

Es gibt nach wie vor zahlreiche Krankheiten, deren Behandlung nur in untergeordnetem Maße möglich ist, da hierfür noch keine geeigneten Arzneimittel existieren. Dies trifft beispielsweise auf Viruserkrankungen, wie Herpes simplex, Herpes labialis, Herpes zoster, Varizellen und auch andere bläschenbildende Hauterkrankungen, zu. Bis heute gibt es mit Ausnahme von Lokalanästhetika, die den Juckreiz etwas lindern, hiergegen keine wirksamen Heilmittel. Darüber hinaus sind einige Virusstatika gegen Herpes bekannt, diese zeigen jedoch zum Großteil starke Nebenwirkungen. So sind Neurodermitis oder Psoriasis (Schuppenflechte) bisher nur mit Corticoid-Präparaten behandelbar. Des weiteren sind einige Krankheiten, wie Akne oder Aphthen nur schwer zu behandeln und die Vielzahl der in Apotheken erhältlichen Substanzen und Zusammensetzungen zeigen einen mehr oder minder geringen Erfolg bei der Heilung und verschaffen nur selten Linderung.

Im Stand der Technik sind einige Vorschläge im Zusammenhang mit Chloramin T, dem Tosylchloramid-Natriumsalz, unterbreitet worden, welches insbesondere als Desinfektionsmittel Verwendung findet:

So beschäftigt sich die Offenbarung der DE 39 13 391 A1 mit einem Desinfektions- und Heilmittel, enthaltend eine Kombination von Chloramin T nur in Verbindung mit einem Reduktionsmittel. Dieses Mittel wird insbesondere zur Bekämpfung von Fischkrankheiten bei Aquarien und Nutzfischen, zur Desinfektion von Fischzuchtanlagen und Schwimmbadwasser, aber auch zur Wunddesinfektion bei Menschen, Säugetieren und Vögeln eingesetzt. Die Aufgabe, welche dieser Lehre zugrunde liegt, besteht darin, toxische Begleiterscheinungen von Chloramin T, insbesondere bei Fischen, zu beseitigen. Hierzu wird ein Reduktionsmittel, wie beispielsweise Natriumthiosulfat oder dergleichen, zugesetzt.

Der Stand der Technik nach der DE-A 197 12 565 bezieht sich auf pharmazeutische Zusammensetzungen, die ein Agenz enthalten, welches ohne entsprechende Bestrahlung Singulett-Sauerstoff erzeugen kann. Ein derartiges Agens ist beispielsweise Chloramin T, wobei die Zusammensetzung zum Desinfizieren und/oder Stabilisieren von Blut, Plasma sowie Blutproben und insbesondere gegen HIV-Erkrankungen eingesetzt wird.

Ferner beschäftigt sich die US-A 3,317,540 mit antimikrobiellen Verbindungen, die sowohl gegen *Staphylococcus aureus* als auch *Salmonella typhosa* wirksam sind. Unter anderem wird auch N-chloro-toluenesulfonamid beziehungsweise dessen Alkalisalz genannt. Diese Verbindungen werden zur Behandlung von Textilien, Kunststoffen und Papier, als Antiseptika sowie Desinfektionsmittel eingesetzt.

Nach der Lehre der DE 41 37 544 C2 wird eine antimikrobielle Wirkstoffkombination zur Antiseptik und Desinfektion von Haut, Schleimhaut und Wunden beschrieben, wobei ein Mehrstoffgemisch mit 0,025 bis 3 % einer sauerstoffabspaltenden und 0,01 bis 3 % einer chlorabspaltenden Verbindung, zusätzlich Harnstoff, Allantoin, Panthenol und/oder Milchsäure enthalten sind. Die sauerstoffabspaltende Verbindung kann ein anorganisches oder organisches Peroxid, Hydroperoxid, eine Peroxysäure oder deren Salz sein, während die chlorabspaltende Verbindung Natriumhypochlorit oder Tosylchloramidnatrium darstellt. Die Aufgabe dieser technischen Lehre besteht insbesondere darin, die geringe Wirksamkeit von sauerstoffabspaltenden Verbindungen, wie beispielsweise Wasserstoffperoxid, als Desinfektionsmittel zu verstärken, was durch Zugabe der obigen Verbindungen erreicht wird.

Der oben geschilderte Stand der Technik ist insofern nachteilig, da Kombinationspräparate aus unterschiedlichen Verbindungen eingesetzt werden, wobei die Mengen und Wirkungsweisen der verschiedenen Bestandteile aufeinander abgestimmt sein müssen. Auch steht praktisch ausschließlich die Desinfektionswirkung der Präparate im Vordergrund, womit häufig eine hautreizende Wirkung verbunden ist. Im Stand der Technik nach der WO 91/07876 A1 wird ein Mittel gegen Retroviren, insbesondere gegen den HIV-Virus offenbart. Dieses wird auf Gegenständen, wie Plastikmaterialien, medizinischen Instrumenten und dergleichen, aufgebracht, um die Übertragung einer Infektion zu verhindern. Ferner wird auch die Verwendung einer therapeutischen Zusammensetzung gegen Retroviren bereitgestellt. Das Mittel kann hierbei Chloramin T enthalten. Die bereitgestellte therapeutische Zusammensetzung verhindert die Aktivierung von lymphozytären Zellen, wo die Replikation des Virus erfolgt, d.h. die Multiplikation von HIV-Viren findet nicht statt. Retroviren unterscheiden sich grundsätzlich von den erfindungsgemäß zu behandelnden DNA-Viren, wie beispielsweise Herpes-Viren. So sind Retroviren umhüllte Viren mit einem Durchmesser von 80 bis 120 nm, wobei ihr Genom aus 2 RNA-Molekülen besteht, einzelstrangig und positivstrangorientiert ist und die Länge zwischen 8 bis 11 kB variiert. Zudem weist das Genom dieser Viren spezielle Besonderheiten auf, wodurch sich diese Viren von anderen Viren grundlegend unterscheiden.

Der Erfindung liegt demnach die Aufgabe zugrunde, die Präparate nach dem eingangs beschriebenem Stand der Technik so weiterzubilden, dass eine weitere Behandlungsmöglichkeit von Erkrankungen, insbesondere Haut- und Schleimhauterkrankungen, zur Verfügung gestellt wird, welche praktisch keine Nebenwirkungen aufweist. Das Behandlungsverfahren sollte in einfacher Weise erfolgen, und über möglichst viele Verabreichungswege angewendet werden können. Ferner sollte die Behandlung ein breites Wirkungsspektrum haben und eine möglichst große Bandbreite an Erkrankungen erfassen. Es sollte auch keine Einschränkung der Behandlung auf eine bestimmte Darreichungsform vorliegen. Darüber hinaus sollte die bereitgestellte Zubereitung die auftretenden Erkrankungen direkt lindern und dann heilen.

Erfindungsgemäß wird obige Aufgabe gelöst durch die Verwendung von Tosylchloramid(en), Tosylchloramidsalz(en), deren Derivaten und/oder Abbauprodukten zur Herstellung von Arzneimitteln zur Behandlung von Erkrankungen der Haut und der Schleimhaut, ausgenommen die Behandlung von Retrovirus-(HIV)-Erkrankungen und die Desinfektion.

Somit können Tosylchloramid, dessen Salze, Derivate sowie Abbauprodukte sowohl einzeln als auch in Kombination eingesetzt werden. Die behandelbaren Erkrankungen umfassen zum Beispiel Viruserkrankungen, wie Herpes genitalis: die wohl am weitesten verbreitete Geschlechtskrankheit, Herpes simplex, Herpes labialis, Windpocken: Zoster-Varizellen-Virus, Gürtel- und Gesichtsrose: Herpes zoster, Juckreiz der Haut, wie Mückenstiche und auch bläschenbildende Haut-, Schleimhaut- und Gewebeerkrankungen sowie Neurodermitis, Psoriasis, Akne , Aphten, Stomatitis aphtosa und -herpetica.

In einer besonders bevorzugten Ausführungsform der Erfindung können Haut- und Schleimhauterkrankungen behandelt werden, insbesondere solche die zu Effloreszenzen führen oder führen können. Unter "Effloreszenzen" versteht man Formen krankhafter Hautveränderungen. Beispielsweise können dies unmittelbar durch eine Krankheit hervorgerufene sogenannte Primäreffloreszenzen sein. Dies sind zum Beispiel: Flecken, Knötchen, oberflächliche oder tiefer liegende Knoten, Knollen, Tumore, Geschwülste, Quaddeln, aufgeweitete oberflächlich verlaufende Blutgefäße, Bläschen, wie Eiterbläschen, Blasen und Zysten. Sogenannte Sekundäreffloreszenzen entwickeln sich im Anschluss an die primären Effloreszenzen und äußern sich in Schuppen, Krusten, Erosionen, Abschürfungen, Schrunden, Geschwüren, Narben, Hautschwund und -verdickungen.

Derartige Krankheitsbilder können durch die erfindungsgemäße Zusammensetzung überraschenderweise unmittelbar gelindert und in der Regel geheilt werden. Die Linderung kann beispielsweise darin bestehen, dass bei Haut-, Schleimhaut-, Gewebe- oder Organerkrankungen der damit verbundene starke Juckreiz verschwindet.

Die zu behandelnden Erkrankungen mit oder ohne Effloreszenzen können durch Mikroorganismen hervorgerufen und/oder von Mikroorganismen begleitet sein oder auf parasitäre Einwirkungen zurückgehen. Unter Mikroorganismen werden im Rahmen der Erfindung sowohl Bakterien, Viren (außer Retroviren), Myzeten als auch Zooparasiten verstanden, die hier ebenfalls miteinbezogen sind. Zum Beispiel schließt dies Krankheiten, wie Skabies, Pediculosis oder Creeping Eruption, ein. Insbesondere können mit der erfindungsgemäß verwendeten Zubereitung behandelt werden: a) das Auge, insbesondere Lid, Bindehaut oder Hornhaut des Auges; b) das Ohr, insbesondere das Äußere des Ohrs; c) die Nase, insbesondere die Nasenhöhle; d) die Lippen und Schleimhaut des Mundes, und/oder die Zunge; e) die Vulva und/oder Vagina; f) der Penis, insbesondere die Glans penis und die Vorhaut; g) der Anus; h) der Nagel, insbesondere das Nagelbett, der Nagelwall und -falz sowie die Nagelwurzel; i) das Haar, insbesondere die Haarfollikel und die Talgdrüsen und j) die Hände und die Füße, insbesondere Finger- und Zehenzwischenräume.

Nach einer bevorzugten Aussführungsform der Erfindung werden Tosylchloramidsalze, insbesondere als Alkali- und Erdalkalisalze eingesetzt. Vorzugsweise der Einsatz von Natrium- und Calcium- als auch Kaliumsalzen führt zu hervorragenden Ergebnissen bei der Behandlung. Insbesondere bevorzugt ist das Natriumsalz, welches unter dem Handelsnamen Chloramin T von der Firma Synopharm aus 22882 Barsbüttel vertrieben wird.

Vorzugsweise findet der Tosylchloramid-Wirkstoff in Form von Tosylchloramid, Salz, Derivat und/oder Abbauprodukt, einzeln oder in Kombination, in einer Menge von etwa 0,1 bis 20 Gew.-%, bevorzugt etwa 5 bis 15 Gew.-%, insbesondere etwa 8 bis 12 Gew.-% Verwendung, je nach der eingesetzten Grundlage.

"Grundlage" bedeutet im Rahmen der vorliegenden Erfindung die Darreichungsform der Zubereitung, die erfindungsgemäß nicht besonders beschränkt ist. Es kann sich hierbei um flüssige, halbfeste oder feste, wasserhaltige oder wasserfreie galenische Zubereitungen, wie Salben, Gele, Cremen, Pasten, Zäpfchen, Tabletten, Brausetabletten, Kapseln, Stifte, wie Lippenstifte, Pulver, Puder, Lösungen, Pflaster, Aerosole, Zweikompartimentsysteme oder Suspensionen wie z.B. Schüttelmixturen/Trockensuspensionen, handeln.

Ist die Grundlage ein Gel, liegt der Tosylchloramid-Wirkstoff in einer Menge von etwa 0,1 bis 5 Gew.-%, insbesondere etwa 0,1 bis 2 Gew.-% vor. Bei den Gelen kann es sich um Kohlenwasserstoffgele handeln. Kohlenwasserstoffgele sind wasserfreie Grundlagen, die sich durch weitgehende chemische Indifferenz und hohe Haltbarkeit auszeichnen. Ein Zusatz von Konservierungsmitteln ist nicht erforderlich. Vorzugsweise wird Vaseline als Grundlage eingesetzt, ihre Konsistenz läßt sich durch feste oder flüssige Paraffine, Wachse und Fettalkohole beliebig verändern. Kohlenwasserstoffgele decken die behandelten Hautareale feuchtigkeitsdurchlässig ab, wodurch die Mazeration des Stratum corneum bewirkt wird. Die enthaltenen Arzneistoffe können dadurch in der Regel in tiefere Hautschichten penetrieren. Die Anwendung ist daher im chronischen Stadium verschiedener Dermatosen angezeigt. Bekannte Grundlagen für Gele sind auch hochpolymere Polyethylene und flüssiges Paraffin, die sich von Vaseline im Schmelzverhalten unterscheiden und einen deutlich höheren Tropfpunkt aufweisen.

Des weiteren sind Hydrogele als Grundlage geeignet. Hydrogele sind fettfreie, abwaschbare Grundlagen, die durch Gelbildung organischer oder anorganischer Hilfsstoffe mit hohen Mengenanteilen Wasser (etwa 90 bis 98%) hergestellt werden. In der Regel enthalten sie ferner Feuchthaltemittel und Konservierungsmittel. Als organische Gelbildner können anionische Verbindungen, wie Carboxymethylcellulose und Alginate, oder nichtionische Makromoleküle, wie Methylcellulose und Hydroxyethylcellulose, herangezogen werden. Derartige Hydrogele wirken zunächst kühlend und bilden nach Verdunsten des Wassers auf der Hautoberfläche abdeckende, antrocknende Filme. Im Gegensatz dazu werden mit anionischen Polyacrylaten vom Carbopol-Typ Hydrogele erhalten, welche sich in die Haut einreiben lassen und deshalb eine gewisse Tiefenwirkung besitzen. Bei Neutralisation der Polyacrylsäure mit bestimmten Aminen lassen sich mit Ethanol oder Isopropanol alkoholhaltige Gele herstellen, die sowohl den Kühleffekt als auch die Tiefenwirkung zu verstärken vermögen. Hydrogele eigen sich vorzugsweise zur Behandlung von Neurodermitis, Psoriasis, Akne, Mückenstichen und Aphten.

Als Salben, wie z.B. Haft- oder Augensalben, werden vorzugsweise W/O-Emulsionssalben eingesetzt. Aufgrund ihrer lipophilen Außenphase sind W/O-Emulsionssalben auf der Haut fettend und nicht mit Wasser abwaschbar. Sie enthalten einen oder mehrere W/O-Emulgatoren, wie z.B. Wollwachs, Wollwachsalkohol, höhere Fettalkohole oder Glycerinfettsäureester. W/O-Emulsionssalben können konserviert werden, häufig sind Antioxidantien enthalten. Sie weisen ein gutes Spreitvermögen auf der Haut auf. Im Gegensatz zu O/W-Emulsionen ist die Strömungsrichtung der Hautfeuchtigkeit unter dem Einfluß der lipophilen Außenphase dieses Salbentyps in das Innere der Haut gerichtet. Dadurch werden Quellung und Penetration verstärkt. Diese Grundlagen eigenen sich vorzugsweise für trockene Haut.

Ein anderer geeigneter Salbentyp sind die O/W-Emulsionssalben. O/W-Emulsionssalben (Cremes) sind aufgrund ihrer wäßrigen Außenphase abwaschbar. In der Regel enthalten sie Komplexemulgatoren, deren hydrophile Komponente von Fettalkohofsulfaten oder nichtionischen polyethylenglykolhaltigen Tensiden gebildet wird. Als lipophile Stabilisatoren werden Fettalkohole, Sorbitanfettsäureester oder Glycerinfettsäureester eingesetzt. O/W-Emulsionen enthalten häufig Feuchthaltemittel und müssen in der Regel mit Konservierungsmitteln stabilisiert werden. Bedingt durch ihre wäßrige Außenphase lassen sich Cremes im allgemeinen gut auf der Haut verteilen und besitzen einen Kühleffekt. O/W-Emulsionssalben werden zur Behandlung subakuter und subchronischer Dermatosen herangezogen und eigenen sich bevorzugt für den seborrhoeischen Hauttyp. Die O/W- oder W/O-Emulsionssalben eigenen sich besonders bei der Behandlung von Herpes simplex, Herpes labialis, Herpes zoster, Varizellen und sonstigen bläschenbildenden Hauterkrankungen. Der(Die) Tosylchloramid-Wirkstoff(e) ist(sind) in O/W- bzw. W/O-Salben in einer Menge von vorzugsweise etwa 0,1 bis 20 Gew.-%, besonders bevorzugt etwa 5 bis 15 Gew.-% und insbesondere etwa 8 bis 12 Gew.-% enthalten.

Eine besonders bevorzugte Zubereitung enthält 10 Gew.-% Tosylchloramid-Wirkstoff(e) in einer cortisonhaltigen Haftsalbe (0,001 Gew.-% Corticoid), wie Volon A®, einem Handelsnamen der Firma Squibb-Heyden GmbH. In dieser Zubereitung ist der Tosylchloramid-Wirkstoff besonders effektiv, vorzugsweise bei Herpes simplex, Herpes labialis, Herpes zoster, Varizellen und sonstigen bläschenbildenden Hauterkrankungen, sowie Aphten, Stomatitis aphtosa und - herpetica.

Weitere mögliche Grundlagen sind feste galenische Zubereitungen, wie Puder, Pulver oder Pasten, die beispielsweise als Badezusatz für Hand-, Fuß- oder Vollbäder Verwendung finden können und den oder die Tosylchloramid-Wirkstoffe in einer Menge von etwa 0,1 bis 20 Gew.-% enthalten. Bei Einsatz eines Pulvers enthält das Badewasser bevorzugt eine Konzentration von etwa 0,1 bis 1 Gew.-% an Zusatz (insbesondere bei Neurodermitis). Feste Zubereitungen umfassen ferner Zäpfchen oder Vaginalkugeln, die sich auflösen oder einen auskleidenden Schaum bilden, Stifte zur Behandlung von Lippen, Haut- oder Schleimhautarealen (insbesondere bei Herpes labialis), Pflaster, zum Beispiel mit Gewebe natürlichen oder künstlichen Ursprungs oder beidem, unversiegelt oder luft- und wasserdampfdicht versiegelt, Tabletten, wie Kapseln aus Weich- oder Hartgelatine, Brausetabletten und dergleichen.

Ferner können auch Aerosole, wie beispielsweise Sprays, in gasdichten Behältern eingesetzt werden, die mechanisch zu bedienen sind oder mit FCKW-freiem Treibgas vorliegen, wie zum Beispiel ein Sprühnebel, Schaum, Gel oder Pflaster, der auf den zu behandlenden Haut- oder Schleimhautpartien erzeugt wird. Insbesondere kommen hierfür auch Dosieraerosole oder Dosierlösungen in Betracht, die pro Hub bzw. Sprühstoß eine definierte Menge an Substanz freisetzen. Ebenfalls können Zweikomponentensysteme in gasdichten Behältnissen eingesetzt werden, bei denen der oder die Tosylchloramid-Wirkstoffe allein oder in einem inerten Trägermaterial und/oder Treibmittel in zwei getrennten Kammern aufbewahrt und erst beim jeweiligen Gebrauch miteinander gemischt werden.

Die mit der Erfindung verbundenen Vorteile sind vielschichtig. So hat sich gezeigt, dass Tosylchloramid-Verbindungen bei allen bläschenbildenden, juckenden, viral bedingten Haut- und Schleimhauterkrankungen einsetzbar sind. Es wird nicht nur eine schnelle Linderung der akuten Symptomatik, sondern auch eine Abnahme der Rezidivhäufigkeit erzielt.

Besonders überraschend ist, dass die erfindungsgemäße Verwendung des Tosylchloramid-Wirkstoffs zur Herstellung von Arzneimitteln völlig unabhängig von der eingesetzten Grundlage zu einem sehr guten Behandlungsergebnis führt, es liegt keine Festlegung auf einen bestimmten Verabreichungsweg vor. Hierbei können bereits relativ geringe Mengen des Tosylchloramid-Wirkstoffs zu einer vollständigen Abheilung führen. Darüber hinaus kann, wie oben dargelegt, die Applikationsform den speziellen Bedürfnissen angepasst werden, so dass eine große Variabilität der Darreichungsform möglich ist.

Die direkte bzw. lokale Applikation, beispielsweise in Form von Gelen, Salben, Stiften oder als Badezusatz hat die Vorteile, dass die betroffene Stelle unmittelbar behandelt werden kann, auch bei feuchter Schleimhaut eine gute Haftung erzielt und gleichzeitig eine lange Verweildauer und Einwirkzeit erreicht werden kann. Insbesondere von Vorteil ist auch die galenische Form des Sprays, wobei durch Besprühen von schmerzenden oder juckenden Hautpartien die Applikation des Wirkstoffs im Gegensatz zum Auftragen einer Salbe schmerz- und berührungsfrei erfolgen kann, was die compliance eines Patienten sicherlich erhöht. Die lokale Anwendung führt zunächst nach einem spürbaren Brennen nach dem Auftragen zu einer raschen Linderung der akuten Beschwerden, die Abheilung eventuell vorhandener Effloreszensen erfolgt in der Regel binnen weniger Tage. Die Behandlung kann auch bereits vor Entstehen etwaiger Effloreszenzen erfolgen, die erst im letzten Stadium einer Hauterkrankung gebildet werden. Gerade im Falle von Herpes werden derartige Effloreszenzen durch die Behandlung vermieden, so dass auch eine vorbeugende Behandlung möglich ist.

Tosylchloramid-Verbindungen, wie Chloramin T, sind seit langem bekannte Desinfektionsmittel, die auch zur Trinkwasserdesinfektion verwendet werden und somit ausgiebig auf ihre Wirkungen, ihr Eliminationsverhalten, unerwünschte Wirkungen, Gegenanzeigen, Wechselwirkungen, toxikologischen Eigenschaften sowie Mutagenität usw. untersucht. Insbesondere die Verträglichkeit bei direktem Kontakt mit der Haut sowie die Sensibilisierungspotenz lassen daher die erfindungsgemäßen Verwendungen, wie beispielsweise die lokale Anwendung in entsprechender Konzentration in Verbindung mit einer Salbe, unbedenklich erscheinen.

### Beispiele:

Die nachfolgenden Beispiele 1 bis 3 zeigen die Herstellung einiger erfindungsgemäß eingesetzter Grundlagen im Einzelnen. Im Anschluss wird in den Beispielen 4 bis 7 die Behandlung des Tosylchloramid-Wirkstoffs aufgezeigt.

### Beispiel 1:

### Herstellung einer W/O-Emulsionssalbe in Form einer Wollwachsalkoholsalbe

Die Wollwachsalkoholsalbe hatte folgende Zusammensetzung:

| | |
|---|---|
| Wollwachsalkohole | 6,0 Teile |
| Cetylstearylalkohol | 0,5 Teile |
| Weißes Vaselin | 93,5 Teile |

Die Substanzen wurden auf dem Wasserbad geschmolzen und bis zum Erkalten gerührt. Bis zu 12 Teile des Vaselins können durch dickflüssiges Paraffin ersetzt sein. Mit dieser Salbe wurde nachfolgende Zusammensetzung hergestellt:

| | |
|---|---|
| Wollwachsalkohlsalbe | 1 Teil |
| Wasser | 1 Teil |
| Tosylchloramid-Natrium | 0,2 Teile |

In die auf etwa 60°C erwärmte Wollwachsalkoholsalbe wurde das auf gleiche Temperatur erwärmte Wasser eingearbeitet. Die Salbe wurde bis zum Erkalten gerührt und das Tosylchloramid-Natrium eingearbeitet.

### Beispiel 2:

### Herstellung einer O/W-Emulsionsalbe in Form einer hydrophilen Salbe

Die hydrophile Salbe hatte folgende Zusammensetzung:

| | |
|---|---|
| Emulgierender Cetylstearylalkohol | 30 Teile |
| Dickflüssiges Paraffin | 35 Teile |
| Weißes Vaselin | 35 Teile |

Die Substanzen wurden auf dem Wasserbad geschmolzen und bis zum Erkalten gerührt. Falls nach der angegeben Vorschrift keine gut streichbare Salbe erhalten wird, dürfen dickflüssiges Paraffin und weißes Vaselin nach Bedarf bis zu 10 Prozent gegeneinander ausgetauscht werden. Mit dieser Salbe wurde nachfolgende Zusammensetzung hergestellt:

| | |
|---|---|
| Hydrophile Salbe | 30 Teile |
| Wasser | 70 Teile |
| Tosylchloramid-Natrium | 14 Teile |

Die hydrophile Salbe wurde auf dem Wasserbad bei etwa 70°C geschmolzen und die Schmelze mit dem auf gleiche Temperatur erwärmten Wasser in kleinen Anteilen versetzt. Die Salbe wurde bis zum Erkalten gerührt und das verdampfte Wasser ersetzt. Tosylchloramid-Natrium wurde eingearbeitet.

### Beispiel 3:

Herstellung eines Hydrogels mit folgender Zusammensetzung:

| | |
|---|---|
| Hydroxyethylencellulose 300 | 4,5 g |
| Glycerol 85 % | 30,5 g |
| Gereinigtes Wasser | 65,5 g |
| Tosylchloramid-Natrium | 2,0 g |

Die Hydroxyethylencellulose wurde in dem frisch abgekochten und wieder abgekühlten Wasser, in dem das Tosylchloramid-Natrium gelöst war, unter Rühren gleichmäßig suspendiert. Die Mischung musste so lange quellen, bis ein klares Gel entstanden war. Anschließend wurde das Glycerol eingerührt. Verdunstetes Wasser wurde gegebenenfalls ergänzt.

### Beispiel 4:

Das in Beispiel 3 erhaltene Hydrogel wurde auf Mückenstiche aufgetragen. Dieses ließ den Juckreiz schnell abklingen. Bläschen trockneten innerhalb von 24 bis 36 Stunden aus, wobei die Abheilung der Haut nach weiteren 24 bis 36 Stunden erfolgte.

### Beispiel 5:

In die bekannte Haftsalbe Volon A®, welche Cortikosteroide enthält, wurde Chloramin T gemischt und die nachfolgenden Behandlungen durchgeführt:

### a) Aphthen:

Eine Salbe, enthaltend den erfindungsgemäß verwendeten Tosylchloramid-Wirkstoff wurde auf Aphthen aufgebracht. Nach dem Auftragen trat ein kurzes Brennen auf, danach war kaum noch eine Empfindlichkeit der behandelten Aphthe zu beobachten, wonach ein schnelles Abheilen des Ulcus innerhalb von 1 bis 2 Tagen erfolgte. Die Therapie von Aphthen zeigte zudem, dass im Gegensatz zu der üblicherweise eingesetzten Zovirax®-Salbe die Anwendung auf der Schleimhaut (Mund, Genitalbereich) möglich und erfolgreich ist.

### b) Herpes labialis:

Der ausgebrochene Herpes heilte in kürzester Zeit ab, was sich durch Kribbeln bemerkbar machte. Bei rechtzeitiger Erkennung kommt der Herpes in der Regel gar nicht zum Ausbruch.

### c) Mundwinkelrhagaden:

Der Verlauf der Behandlung entspricht dem unter 'Herpes labialis' Beschriebenen. Auch hier wurde ein sehr schnelles, dauerhaftes Abheilen beobachtet. Im allgemeinen wird bei Herpesinfektionen in der Mundhöhle, insbesondere am harten Gaumen, ein ausgezeichnetes Behandlungsergebnis mit der erfindungsgemäß verwendeten Haftsalbe erzielt.

### d) Neurodermitis mit Herpes-Superinfektion:

Ein sofortiger Rückgang des unerträglichen Juckreizes bei gleichzeitig verblüffend schneller Abheilung der Haut wurde erreicht. Für Neurodermitiker ist allein das Ausschalten des Juckreizes von allerhöchster Bedeutung, da hiermit das unwiderstehliche Kratzbedürfnis entfällt, das oftmals zu einer Verschlimmerung und weiteren Ausbreitung des Ausschlags führt. Dies kann mit den erfindungsgemäß verwendeten Wirkstoffen erzielt werden.

### Beispiel 6:

Statt der Haftsalbe Volon A® mit Chloramin T-Zusatz wurde eine normale Kühlsalbe 'Unguentum leniens' zubereitet, welche als Wirkstoff lediglich 2 Gew.-% Chloramin T-Pulver enthielt, aber kein Cortikoid. Der unter Beipiel 5 geschilderte Behandlungserfolg war derselbe. Auch die Verwendung einer Hydrolotion, enthaltend nur Chloramin T als Wirkungskomponente, führte zu den oben dargestellten Ergebnissen. Selbst der Zusatz des Pulvers ins Badewasser brachte vergleichbare Ergebnisse.

### Beispiel 7:

Es wurden 15 Probanden mit verschiedenen Krankheitsbildern unter ärztlicher Aufsicht behandelt. Nach der Behandlung über mehrere Tage wurden vom behandelnden Arzt Bewertungen hinsichtlich der Wirkung sowie des Gesamtergebnisses der Behandlung durchgeführt. Für die Beurteilung der Wirkung konnten die folgenden Bewertungen angekreuzt werde: keine - geringfügig - gut - sehr gut - ausgezeichnet. Zur Beurteilung des Gesamtergebnisses der Anwendung waren folgende Beurteilungen möglich: keine Besserung - mäßige Besserung - gute Besserung - sehr gute Besserung. Die vorgenommene Untersuchungen mit den jeweiligen Krankheitsbildern und Ergebnissen sind in der nachfolgenden Tabelle zusammengefaßt:

**Tabelle**

| Proband | Krankheitsbild | Grundlage | Anwendungszeitraum | Beurteilung der Wirkung | Gesamtergebnis der Anwendung |
|---|---|---|---|---|---|
| 1 | Herpes labialis | Haftsalbe Volon A® mit Chloramin T | 5 Tage, 2 mal täglich | Sehr gut | Sehr gute Besserung |
| 2 | Herpes labialis | Haftsalbe Volon A® mit Chloramin T | 3 Tage, 1-3mal täglich | Ausgezeichnet | Sehr gute Besserung |
| 3 | Herpes labialis | Haftsalbe Volon A® mit Chloramin T | 2 Tage 1-2mal täglich | Ausgezeichnet | Sehr gute Besserung |
| 4 | Herpes labialis | Haftsalbe Volon A® mit Chloramin T | 6 Tage 1-4mal täglich | Gut | Gute Besserung |
| 5 | Herpes labialis | Haftsalbe Volon A® mit Chloramin T | 4 Tage 1-3mal täglich | Sehr gut | Gute Besserung |
| 6 | Herpes labialis | Haftsalbe Volon A® mit Chloramin T | 2 Tage 1-2mal täglich | Ausgezeichnet | Sehr gute Besserung |
| 7 | Aphthen | Haftsalbe Volon A® mit Chloramin T | 2 Tage 2-3mal täglich | Ausgezeichnet | Sehr gute Besserung |
| 8 | Psoriasis | Salbe mit 2 Gew.-% Chloramin T | 7 Tage 2-3mal täglich | Geringfügig | Mäßige Besserung |
| 9 | Bläschen mit wässr. Flüssigkeit | Salbe mit 2 Gew.-% Chloramin T | 7 Tage 3-4mal täglich | Geringfügig | Mäßige Besserung |
| 10 | Schuppenflechte am Kopf | Salbe mit 2 Gew.-% Chloramin T | 7 Tage 3mal täglich | Ausgezeichnet | Sehr gute Besserung |
| 11 | LippenRhagade | Salbe mit 2 Gew.-% Chloramin T | 4 Tage 1-3mal täglich | Ausgezeichnet | Sehr gute Besserung |
| 12 | LippenRhagade | Salbe mit 2 Gew.-% Chloramin T | 5 Tage 2mal täglich | Gut | Sehr gute Besserung |
| 13 | Stomatitis Herpetica | Salbe mit 2 Gew.-% Chloramin T | 5 Tage 1-4mal täglich | Ausgezeichnet | Sehr gute Besserung |
| 14 | Neurodermitis mit Herpes-Superinfektion | Salbe mit 2 Gew.-% Chloramin T | 6 Tage 1-2mal täglich | Ausgezeichnet | Sehr gute Besserung |
| 15 | Gürtelrose | Salbe mit 3 Gew.-% Chloramin T | 20 Tage 1 mal täglich | Ausgezeichnet | Sehr gute Besserung |

Die obigen Ergebnisse zeigen klar die überraschend hohe Wirksamkeit bei der erfindungsgemäßen Verwendung der Tosylchloramid-Verbindung. Selbst Schuppenflechte verschwand nach einer 7 tägigen Behandlung vollständig. Lediglich bei zwei wahrscheinlich chronischen Krankheitsbildern konnte nach einer Behandlungsdauer von 7 Tagen nur eine mäßige Besserung erreicht werden.

## Patentansprüche

1. Verwendung von Tosylchloramid(en), Tosylchloramidsalz(en), deren Derivaten und/oder Abbauprodukten zur Herstellung von Arzneimitteln zur Behandlung von Erkrankungen der Haut und der Schleimhaut, ausgenommen die Behandlung von Retrovirus-(HIV)-Erkrankungen und Desinfektion.

2. Verwendung nach Anspruch 1, **dadurch gekennzeichnet, dass** die Erkrankungen zu Effloreszenzen führen oder führen können.

3. Verwendung nach Anspruch 1 oder 2, **dadurch gekennzeichnet, dass** die Erkrankungen und/oder Effloreszenzen durch Mikroorganismen hervorgerufen und/oder von Mikroorganismen begleitet werden können.

4. Verwendung nach einem der Ansprüche 1 bis 3, **dadurch gekennzeichnet, dass** die Erkrankungen parasitäre Erkrankungen, insbesondere Skabies, Pediculosis oder Creeping Eruption, darstellen.

5. Verwendung nach mindestens einem der vorhergehenden Ansprüche, **dadurch gekennzeichnet, dass** die Erkrankungen betreffen:
a) das Auge, insbesondere Lid, Bindehaut oder Hornhaut des Auges;
b) das Ohr, insbesondere das Äußere des Ohrs;
c) die Nase, insbesondere die Nasenhöhle;
d) die Lippen und Schleimhaut des Mundes und/oder die Zunge;
e) die Vulva und/oder Vagina;
f) den Penis, insbesondere die Glans penis und die Vorhaut;
g) den Anus;
h) den Nagel, insbesondere das Nagelbett, den Nagelwall und -falz sowie die Nagelwurzel;
i) das Haar, insbesondere die Haarfollikel und die Talgdrüsen und
j) die Hände und die Füße, insbesondere Finger- und Zehenzwischenräume.

6. Verwendung nach mindestens einem der vorhergehenden Ansprüche, **dadurch gekennzeichnet, dass** das oder die Tosylchloramide, Tosylchloramidsalze, deren Derivate und/oder Abbauprodukte in einer Menge von etwa 0,1 bis 20 Gew.-%, bevorzugt etwa 5 bis 15 Gew.-%, insbesondere etwa 8 bis 12 Gew.-% in der eingesetzten Grundlage vorliegen.

7. Verwendung nach mindestens einem der vorhergehenden Ansprüche, **dadurch gekennzeichnet, dass** ein Tosylchloramidsalz, insbesondere Chloramin T, eingesetzt wird.

8. Verwendung nach mindestens einem der vorhergehenden Ansprüche, **dadurch gekennzeichnet, dass** die Grundlage eine flüssige, halbfeste oder feste, wasserhaltige oder wasserfreie galenische Zubereitung darstellt.

9. Verwendung nach Anspruch 8, **dadurch gekennzeichnet, dass** die Grundlage eine Salbe, ein Gel, eine Creme, eine Paste, ein Zäpfchen, wie ein Vaginalzäpfchen, ein Pflaster, eine Tablette, wie eine Brausetablette oder Vaginaltablette, oder eine Kapsel, einen Stift, ein Pulver, einen Puder, eine Lösung, ein Aerosol, ein Zweikompartimentsystem oder eine Suspension, wie eine Schüttelmixtur/Trockensuspension darstellt.

10. Verwendung nach Anspruch 8 oder 9, **dadurch gekennzeichnet, dass** die Grundlage ein Dosieraerosol oder eine Dosierlösung darstellt.

11. Verwendung nach Anspruch 8 oder 9, **dadurch gekennzeichnet dass** die Grundlage einen Badezusatz darstellt.

12. Verwendung nach Anspruch 8 oder 9, **dadurch gekennzeichnet, dass** die Salbe eine O/W- oder eine W/O-Emulsionssalbe ist.

13. Verwendung nach mindestens einem der vorhergehenden Ansprüche, **dadurch gekennzeichnet, dass** die Grundlage eine cortisonhaltige Zubereitung ist, die das oder die Tosylchloramide, Tosylchloramidsalze, deren Derivate und/oder Abbauprodukte in einer Menge von etwa 0,1 bis 20 Gew.-% enthält.

14. Verwendung nach Anspruch 8 oder 9, **dadurch gekennzeichnet, dass** die Grundlage ein Gel ist, indem das oder die Tosylchloramide, Tosylchloramidsalze, deren Derivate und/oder Abbauprodukte in einer Menge von etwa 0,1 bis 5 Gew.-%, insbesondere etwa 0,1 bis 2 Gew.-% vorliegen.

15. Verwendung nach Anspruch 8 oder 9, **dadurch gekennzeichnet, dass** der Badezusatz als Pulver oder Badesalztablette oder Brausetablette eingesetzt wird, der in einer Konzentration von etwa 0,1 bis 1 Gew.-% in Wasser zur Anwendung kommt.

## Claims

1. The use of tosylchloramide(s), tosylchloramide salt(s), derivatives and/or decomposition products thereof to produce pharmaceutical preparations for the treatment of diseases of the skin and mucus membranes, excluding the treatment of retrovirus (HIV) diseases and disinfection.

2. The use as claimed in claim 1, **characterised in that** the diseases result or can result in efflorescences.

3. The use as claimed in claims 1 or 2, **characterised in that** the diseases and/or efflorescences can be caused by micro-organisms and/or be accompanied by micro-organisms.

4. The use claimed in one of claims 1 to 3, **characterised in that** the diseases constitute parasitic diseases, particularly scabies, pediculosis or creeping eruption.

5. The use as claimed in at least one of the preceding claims, **characterised in that** the diseases relate to:
a) the eye, particularly the lid, conjunctiva or cornea of the eye;
b). the ear, particularly the exterior of the ear;
c) the nose, particularly the nostrils;
d) the lips and mucus membrane of the mouth/or the tongue;
e) the vulva and/or vagina;
f) the penis, particularly the glans penis and the foreskin;
g) the anus;
h) the nails, particularly the nail bed, the nail wall, the nail fold and the nail root;
i) the hair, particularly the hair follicles and the sebaceous glands; and
j) the hands and the feet, particularly the gaps between the fingers and toes.

6. The use as claimed in at least one of the preceding claims, **characterised in that** the tosylchloramide(s), tosylchloramide salt(s) and the derivatives and/or decomposition products thereof are present in an amount of about 0.1 to 20% by wt, preferably by about 5-15% by wt, preferably about 5-15% wt, particularly about 8-12% wt in the base that is used.

7. The use as claimed in at least one of the preceding claims, **characterised in that** a tosylchloramide salt, particularly chloramine T is used.

8. The use as claimed in at least one of the preceding claims, **characterised in that** the base constitutes a liquid, semi-solid or solid, water-containing or water-free galenic preparation.

9. The use as claimed in claim 8, **characterised in that** the base constitutes an ointment, a gel, a cream, a paste, a suppository such as a vaginal suppository, a plaster, a tablet, such as an effervescent tablet or vaginal tablet, or a capsule, a stick, a powder, a dust, a solution, an aerosol, a two-component system or a suspension, such as a vibration mixture/dry suspension.

10. The use as claimed in claims 8 or 9, **characterised in that** the base constitutes a dosing aerosol or a dosing solution.

11. The use as claimed in claims 8 or 9, **characterised in that** the base constitutes a bath additive.

12. The use as claimed in claims 8 or 9, **characterised in that** the ointment is an O/W or W/O emulsion ointment.

13. The use as claimed in at least one of the preceding claims, **characterised in that** the base is a cortisone-containing preparation, which contains the tosylchloramide(s), tosylchloramide salt(s), the derivatives and/or decomposition products thereof in an amount of about 0.1-20% wt.

14. The use as claimed in claims 8 or 9, **characterised in that** the base is a gel, in which the tosylchloramide(s), tosylchloramide salt(s), the derivatives and/or decomposition products thereof are present in an amount of about 0.1-5% wt, particularly about 0.1-2% wt.

15. The use as claimed in claims 8 or 9, **characterised in that** the bath additive is used in the form of a powder or bath salt tablet or effervescent tablet which is used in water in a concentration of about 0.1-1% wt.

## Revendications

1. Utilisation de tosylchloramide(s), de sel(s) de tosylchloramides, de leurs dérivés et/ou de leurs produits de dégradation pour la préparation de médicaments destinés au traitement de maladies de la peau et des muqueuses, excepté le traitement de maladies à rétrovirus (VIH) et la désinfection.

2. Utilisation suivant la revendication 1, **caractérisée en ce que** les maladies conduisent ou peuvent conduire à des efflorescences.

3. Utilisation suivant la revendication 1 ou 2, **caractérisée en ce que** les maladies et/ou les efflorescences peuvent être causées par des microorganismes et/ou associées à des microorganismes.

4. Utilisation suivant l'une des revendications 1 à 3, **caractérisée en ce que** les maladies représentent des maladies parasitaires, en particulier gale, pédiculose ou myiase rampante cutanée.

5. Utilisation suivant au moins l'une des revendications précédentes, **caractérisée en ce que** les maladies concernent :
a) l'oeil, en particulier la paupière, la conjonctive et la cornée de l'oeil ;
b) l'oreille, en particulier l'extérieur de l'oreille ;
c) le nez, en particulier les fosses nasales ;
d) les lèvres et la muqueuse buccale et/ou la langue ;
e) la vulve et/ou le vagin ;
f) le pénis, en particulier le gland et le prépuce ;
g) l'anus ;
h) l'ongle, en particulier le lit unguéal, le repli unguéal et le sillon latéral de l'ongle ainsi que la racine unguéale ;
i) les poils, en particulier les follicules pileux et les glandes sébacées et,
j) les mains et les pieds, en particulier les espaces entre les doigts et les orteils.

6. Utilisation suivant au moins l'une des revendications précédentes, **caractérisée en ce que** le ou les tosylchloramides, le ou les sels de tosylchloramides, leurs dérivés et/ou leurs produits de dégradation sont présents en une quantité d'environ 0,1 à 20% en poids, mieux encore d'environ 5 à 15% en poids, notamment d'environ 8 à 12% en poids dans la base utilisée.

7. Utilisation suivant au moins l'une des revendications précédentes, caractérisée en qu'on utilise un sel de tosylchloramide, en particulier la chloramine T.

8. Utilisation suivant au moins l'une des revendications précédentes, **caractérisée en ce que** la base est une préparation galénique contenant de l'eau ou anhydre, liquide, semi-solide ou solide.

9. Utilisation suivant la revendication 8, **caractérisée en ce que** la base est une pommade, un gel, une crème, une pâte, un suppositoire tel qu'un ovule, un emplâtre, un comprimé tel qu'un comprimé effervescent ou un comprimé vaginal, ou bien une gélule, un crayon, une poudre à pulvériser, une poudre pour poudrage, une solution, un aérosol, un système à deux compartiments ou une suspension, telle qu'une suspension de substituant sèche dans un mélange à agiter.

10. Utilisation suivant la revendication 8 ou 9, **caractérisée en ce que** la base est un aérosol de dosage ou une solution de dosage.

11. Utilisation suivant la revendication 8 ou 9, **caractérisée en ce que** la base est un additif pour le bain.

12. Utilisation suivant la revendication 8 ou 9, **caractérisée en ce que** la pommade est une émulsion huile/eau ou émulsion eau/huile.

13. Utilisation suivant au moins l'une des revendications précédentes, **caractérisée en ce que** la base est une préparation à la cortisone, qui contient le ou les tosylchloramides, le ou les sels de tosylchloramides, leurs dérivés et/ou leurs produits de dégradation en une quantité d'environ 0,1 à 20% en poids.

14. Utilisation suivant la revendication 8 ou 9, **caractérisée en ce que** la base est un gel dans lequel le ou les tosylchloramides, le ou les sels de tosylchloramides, leurs dérivés et/ou leurs produits de dégradation sont présents en une quantité d'environ 0,1 à 5% en poids, notamment d'environ 0,1 à 2% en poids.

15. Utilisation suivant la revendication 8 ou 9, **caractérisée en ce que** l'additif pour le bain est utilisé sous forme de poudre ou de comprimé de sels de bain ou de comprimé effervescent, qui est introduit dans l'eau à une concentration d'environ 0,1 à 1% en poids.
